# EUROPÄISCHE PATENTSCHRIFT

(11) **EP 3 996 614 B1**
(45) Veröffentlichungstag und Bekanntmachung des Hinweises auf die Patenterteilung: **18.10.2023**
(21) Anmeldenummer: 19752912.6
(22) Anmeldetag: 11.07.2019
(51) Int. Cl.: A61B 17/80

(54) **FRAKTURFIXATIONSPLATTE ZUR APPLIKATION AM PROXIMALEN HUMERUS**
FRACTURE FIXATION PLATE FOR APPLICATION TO THE PROXIMAL HUMERUS
PLAQUE DE FIXATION DE FRACTURE POUR L'APPLICATION SUR L'HUMÉRUS PROXIMAL

(43) Veröffentlichungstag der Anmeldung: 18.05.2022
(73) Patentinhaber: Bonebridge AG, 6300 Zug (CH)
(72) Erfinder: SCHALLBERGER, Alex, 6372 Ennetmoos (CH); BURKI, Patrick, 4500 Solothurn (CH); MOOR, Beat Kaspar, 3973 Venthône (CH); GERBER, Christian, 6300 Zug (CH)
(74) Vertreter: Lusuardi, Werther
(86) Internationale Anmeldenummer: PCT/CH2019/000020
(87) Internationale Veröffentlichungsnummer: WO 2021/003583

(56) Entgegenhaltungen:
- EP-A1- 3 178 424
- US-A1- 2008 119 895
- US-A1- 2014 277 177
- US-A1- 2015 327 899
- US-B1- 6 283 969
- US-B2- 7 335 204

## Beschreibung

Die Erfindung bezieht sich auf eine Frakturfixationsplatte zur Applikation am proximalen Humerus gemäss dem Oberbegriff des Patentanspruchs 1.

Die US 6,283,969 Grusin offenbart eine distale Radiusplatte mit zwei seitlichen Flügeln, welche allerdings auf der gleichen Geraden liegen und keinen Winkel zueinander einschliessen. Dadurch wird das korrekte Fassen der Tubercula (majus et minus) verunmöglicht.

Aus der US 2009/0125069 Sixto et al. sowie der US 2018/0250046 Austin et al. sind weitere Knochenfixationsplatten bekannt, welche jedoch folgende Nachteile aufweisen:
- sie sind dem anatomischen und nicht dem frakturierten, respektive reponierten Knochen angepasst; und
- sie sind für die Verwendung am proximalen Humerus nicht optimiert und insbesondere ist der Schaft der Platte zu lang.

Eine Frakturfixationsplatte zur Applikation am proximalen Humerus ist jeweils aus der US 2014/277177 A1, der US 7 335 204 B2 und der US 2008/119895 A1 bekannt.

Hier will die Erfindung Abhilfe schaffen. Der Erfindung liegt die Aufgabe zugrunde, eine Frakturfixationsplatte zur Applikation am proximalen Humerus zu schaffen, welche eine anatomische Reposition und Refixation der Frakturfragmente am proximalen Humerus erlaubt.

Die Erfindung löst die gestellte Aufgabe mit Frakturfixationsplatte, welche die Merkmale gemäss dem Anspruch 1 aufweist.

Die durch die Erfindung erreichten Vorteile sind im Wesentlichen die folgenden:
- Die Tubercula (*Tuberculum majus humeri et tuberculum minus humeri*) können lateral und medial gefasst werden, so dass dadurch ein Absintern der Kalotte verhindert wird; und es wird die Überreposition des Kalkars bei der Reposition durch die Geometrie der Knochenfixationsplatte verhindert.

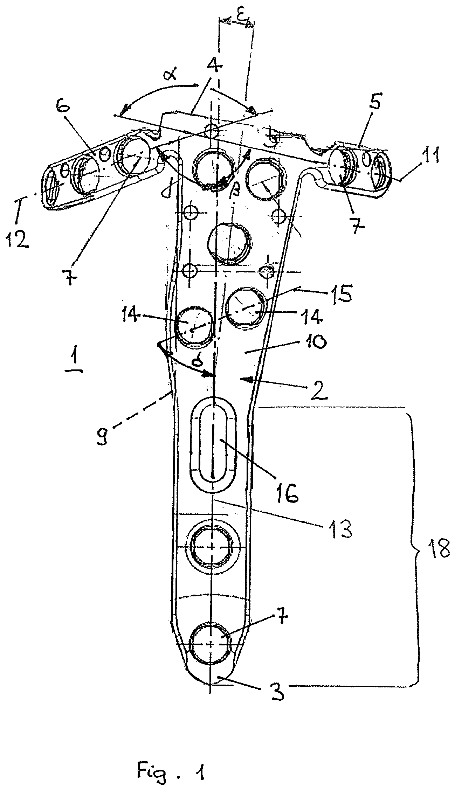

Die Erfindung löst die gestellte Aufgabe mit einer Frakturfixationsplatte gemäss dem Anspruch 1. Weitere vorteilhafte Ausgestaltungen der Erfindung können wie folgt kommentiert werden:
Bei einer besonderen Ausführungsform der Frakturfixationsplatte 1 ist der Winkel α < 175° und liegt vorzugsweise im Bereich von 110° bis 160°. Der Winkel α wird - wie in Fig. 1 dargestellt - zwischen den nach proximal verlängerten Mittellinien 11,12 der beiden Flügel 5,6 gemessen.

Die beiden Flügel 5,6 können kreiszylinderförmig gebogen sein. Dabei können die beiden Flügel 5,6 die gleiche Biegung, vorzugsweise die gleiche kreiszylinderförmige Biegung, aufweisen.

Bei einer weiteren Ausführungsform sind die Mittellinien 11,12 helixförmig gebogen. Der distale Abschnitt 18 des Körperabschnitts 2 kann eine Längsmittellinie 13 definieren, welche die Ebene, in welcher die gebogene Mittellinie 11 des rechten Flügels 5 liegt, unter einem Winkel β ≠ 90° schneidet. Der Winkel β kann vorteilhafterweise im Bereich zwischen 60° und 85° liegen.

Der distale Abschnitt 18 des Körperabschnitts 2 kann eine Längsmittellinie (13) definieren, welche die Ebene, in welcher die gebogene Mittellinie 12 des linken Flügels (6) liegt unter einem Winkel γ ≠ 90° schneidet. Der Winkel γ ≠ 90° liegt vorteilhafterweise im Bereich von 50° bis 80° t.

Bei einer besonderen Ausführungsform ist der linke Flügel 6 stärker abgewinkelt als der rechte Flügel 5, so dass γ < β ist. Diese Ausführungsform hat sich als optimal für eine sichere Fassung des Tuberculum majus und des Tuberculum minus des Schulterkopfes erwiesen.

Das proximale Ende (4) des Körperabschnitts 2 und die beiden Flügel 5,6 können bündig sein. Dadurch kommt das proximale Ende der Knochenfixationsplatte weniger nach distal zu liegen, so dass eine Einklemmung im Schulterdach (Impingement) verhindert wird.

Der langgestreckten Körperabschnitt 2 der Frakturfixationsplatte kann eine Anzahl Gewindelöcher 14 aufweisen, welche gegenüber der Längsmittellinie 13 versetzt angeordnet sind. Diese Ausführungsform hat den Vorteil, dass die Knochenfixationsplatte schmäler wird und sich somit die Auflagefläche auf dem Knochen verringert.

Die Verbindungslinie 15 zwischen den Mittelpunkten zweier solcher versetzt angeordneter Gewindelöcher 14 können die Längsmittellinie 13 unter einem Winkel δ ≠ 90° schneiden. Diese Anordnung der Gewindelöcher erlaubt das Einbringen von Kalkarschrauben bei deutlich kleinerer Breite der Knochenfixationsplatte im Vergleich zum Stand der Technik.

Der Winkel δ liegt vorteilhafterweise im Bereich von 10° bis 70°.

Die Mittelachse eines oder mehrerer der versetzt angeordneten Gewindelöcher 14 können die vom Körperabschnitt 2 gebildete Ebene unter einem Winkel ε ≠ 90° schneiden und der Winkel ε kann vorzugsweise im Bereich von 3° bis 10° liegen.

Bei einer weiteren Ausführungsform kann der Körperabschnitt 2 zusätzlich ein längliches Kompressionsloch 16 aufweisen.

Bei einer besonderen Ausführungsform gehorcht das Verhältnis zwischen der Länge L des linken Flügels 6 und der Länge I des rechten Flügels (5) der Bedingung L ≥ 1,2 I .

Bei einer weiteren Ausführungsform schneidet die Längsmittellinie 13 die Ebene, in welcher die gebogene Mittellinie des linken Flügels liegt, unter einem Winkel β_{LH} ≠ 90°. Der Winkel β_{LH} liegt vorteilhafterweise im Bereich zwischen 60° und 85° .

Bei einer weiteren Ausführungsform schneidet die Längsmittellinie 13 die Ebene, in welcher die gebogene Mittellinie des rechten Flügels liegt unter einem Winkel γ_{LH} ≠ 90° . Der Winkel γ_{LH} ≠ 90° liegt vorteilhafterweise im Bereich von 50° bis 80° .

Bei einer besonderen Ausführungsform gilt die Bedingung γ_{LH} ≤ β_{LH} ist.

Bei einer weiteren Ausführungsform ist die Länge des rechten Flügels grösser als das oder gleich dem 1 ,2-fachen der Länge des linken Flügels.

Die Frakturfixationsplatte 1 gemäss der Erfindung eignet sich insbesondere zur Behandlung von Knochenfrakturen des proximalen Humerus.

Die Erfindung und Weiterbildungen der Erfindung werden im Folgenden anhand der teilweise schematischen Darstellungen mehrerer Ausführungsbeispiele noch näher erläutert.

Es zeigen:
Fig. 1 eine Aufsicht auf die Oberseite einer Ausführungsform der erfindungsgemässen Frakturfixationsplatte für den linken Humerus;
Fig. 2 eine Seitenansicht der in Fig. 1 dargestellten Ausführungsform der erfindungsgemässen Frakturfixationsplatte;
Fig. 3 eine Ansicht von distal nach proximal der in Fig. 1 dargestellten Ausführungsform der erfindungsgemässen Frakturfixationsplatte;
Fig. 4 eine perspektivische Ansicht der in Fig. 1 dargestellten, auf den proximalen, lateralen Humerus aufgelegten Ausführungsform;
Fig. 5 einen Längsschnitt durch die in Fig. 4 dargestellten Frakturfixationsplatte;
Fig. 6 eine seitliche perspektivische Ansicht der in Fig. 5 dargestellten Frakturfixationsplatte nach erfolgter Verschraubung am proximalen Humerus; und
Fig. 7 eine perspektivische Ansicht von kranial der in Fig. 6 dargestellten, verschraubten Frakturfixationsplatte.

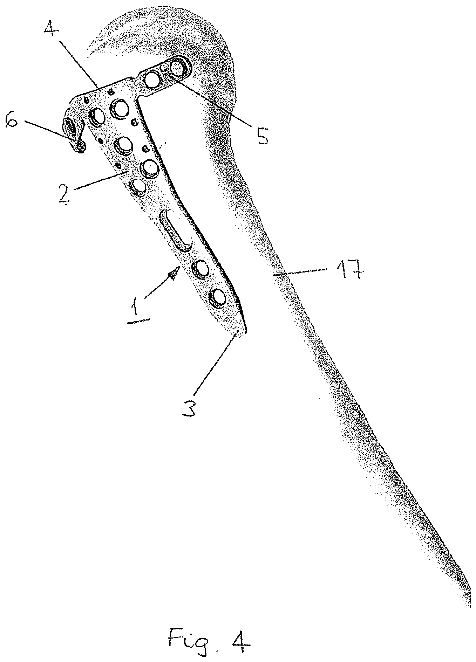

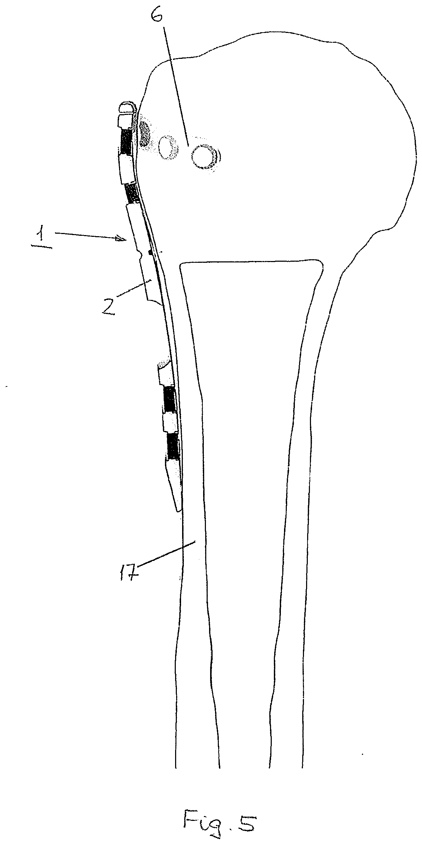

Die in den Fig. 1 bis 7 dargestellte Ausführungsform der erfindungsgemässen Frakturfixationsplatte 1 weist eine Längsmittellinie 13 auf und umfasst im Wesentlichen einen langgestreckten Körperabschnitt 2, welcher ein freies distales Ende 3 und ein proximales Ende 4 aufweist. Der langgestreckte Körperabschnitt 2 umfasst, beispielhaft und nicht einschränkend, einen das proximale Ende 4 umfassenden proximalen Körperabschnitt und einen das distale Ende 3 umfassenden distalen Körperabschnitt, wobei der proximale Körperabschnitt gegenüber dem distalen Körperabschnitt um einen Winkel ε leicht abgewinkelt ist. Ferner schliessen sich am proximalen Ende 4 je seitlich ein rechter Flügel 5 mit der Mittellinie 11 und ein linker Flügel 6 mit der Mittellinie 12. Optimale Werte für die Länge des langgestreckten Körperabschnitts 2 betragen je nach Ausführungsform zwischen 8 - 14 cm.

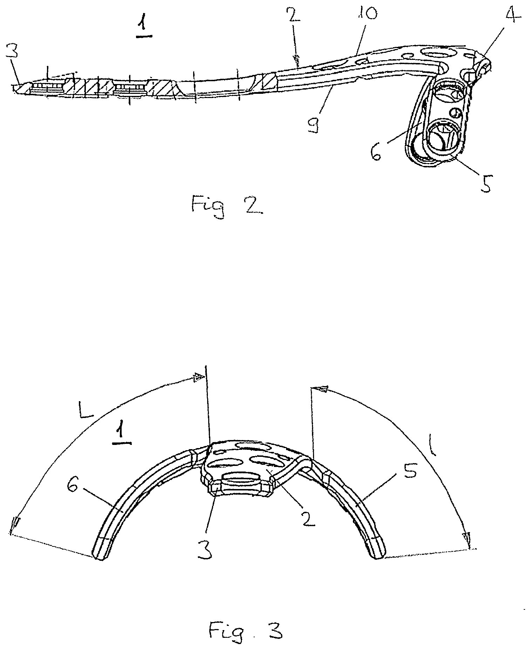

Die beiden Flügel 5,6 weisen eine unterschiedliche Länge auf und sind gebogen, wobei sich die Verlängerungen der beiden gebogenen Mittellinien 11,12 sich unter einem Winkel α schneiden. Der Winkel α beträgt, beispielhaft und nicht einschränkend, zwischen 110° und 160°. Das Verhältnis zwischen der Länge L des linken Flügels 6 (Fig. 3) und der Länge I des rechten Flügels 5 gehorcht der Bedingung L ≥ 1,2 I. Die beiden Flügel 5,6 sind kreiszylinderförmig gebogen, wobei die Mittellinien 11,12 helixförmig gebogen sind. Die beiden Flügel 5,6 weisen, beispielhaft und nicht einschränkend, die gleiche kreiszylinderförmige Biegung auf. Die beiden Flügel 5,6 und das proximale Ende 4 des Körperabschnitts 2 sind bündig.

Die Längsmittellinie 13 schneidet die Ebene, in welcher die gebogene Mittellinie 11 des rechten Flügels 5 liegt, beispielhaft und nicht einschränkend, unter einem Winkel β in einem Bereich zwischen 60° und 85°. Ferner schneidet die Längsmittellinie 13 die Ebene, in welcher die gebogene Mittellinie 12 des linken Flügels 6 liegt, beispielhaft und nicht einschränkend, unter einem Winkel γ zwischen 50° und 80°. Dabei ist der linke Flügel 6 leicht stärker abgewinkelt ist als der rechte Flügel 5, so dass γ ≤ β ist.

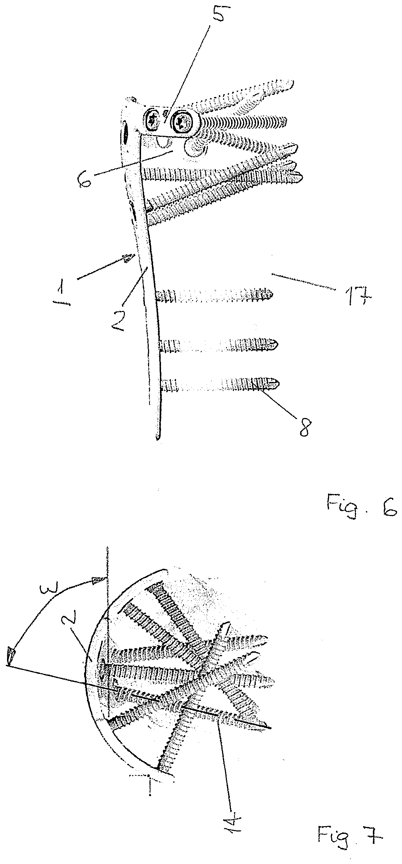

Die Frakturfixationsplatte 1 weist eine Knochenkontaktfläche 9 und eine gegenüberliegende Oberfläche 10 auf. Der Körperabschnitt 2 und die beiden Flügel 5,6 sind mit einer Anzahl Schraubenlöcher 7 zur Aufnahme von Knochenbefestigungselementen 8 (Fig. 6) versehen.

Ferner umfasst der langgestreckten Körperabschnitt 2, beispielhaft und nicht einschränkend, fünf Gewindelöcher 14 auf, welche gegenüber der Längsmittellinie 13 versetzt angeordnet sind, wobei die Verbindungslinie 15 zwischen den Mittelpunkten zweier solcher versetzt angeordneter Gewindelöcher 14 die Längsmittellinie 13, beispielhaft und nicht einschränkend, unter einem Winkel δ zwischen 10° und 70° schneidet. Beispielhaft und nicht einschränkend schneidet die Mittelachse dieser zwei versetzt angeordneten Gewindelöcher 14 die vom Körperabschnitts 2 gebildete Ebene unter einem Winkel ε ≠ 90°. Zusätzlich umfasst der Körperabschnitt 2 ein längliches Kompressionsloch 16.

In den Fig. 1 bis 7 ist eine Ausführungsform für den linken Humerus dargestellt. Für den rechten Humerus sind spiegelbildliche Ausführungsformen vorgesehen, wobei sich eine Ausführungsform für den rechten Humerus nur darin von der in den Fig. 1 bis 7 dargestellten Ausführungsform für den linken Humerus unterscheidet, dass:
- das Verhältnis zwischen der Länge L_{LH} des linken Flügels und der Länge I_{LH} des rechten Flügels der Bedingung L_{LH} ≤ 1,2 I_{LH} gehorcht;
- die Längsmittellinie die Ebene, in welcher die gebogene Mittellinie des linken Flügels liegt, beispielhaft und nicht einschränkend, unter einem Winkel β_{LH} schneidet, welcher in einem Bereich zwischen 60° und 85° liegt;
- die Längsmittellinie die Ebene, in welcher die gebogene Mittellinie des rechten Flügels liegt, beispielhaft und nicht einschränkend, unter einem Winkel γ_{LH} schneidet, welcher zwischen 50° und 80° beträgt; und
- der rechte Flügel stärker abgewinkelt ist als der linke Flügel.

## Patentansprüche

1. Frakturfixationsplatte (1) zur Applikation auf dem proximalen Humerus, wobei
a) die Frakturfixationsplatte (1) einen langgestreckten Körperabschnitt (2) aufweist, welcher ein freies distales Ende (3) und ein proximales Ende (4) aufweist, an welchem sich je seitlich ein rechter Flügel (5) mit der Mittellinie (11) und ein linker Flügel (6) mit der Mittellinie (12) anschliesst;
b) der Körperabschnitt (2) und die beiden Flügel (5,6) mit einer Anzahl Schraubenlöcher (7) zur Aufnahme von Knochenbefestigungselementen (8) versehen sind und die Frakturfixationsplatte (1) eine Knochenkontaktfläche (9) und eine gegenüberliegende Oberfläche (10) aufweist, und
c) die beiden Flügel (5,6) eine unterschiedliche Länge aufweisen,
**dadurch gekennzeichnet, dass**
d) die beiden Flügel (5,6) gebogen sind; und
e) die Verlängerungen der beiden gebogenen Mittellinien (11,12) einen stumpfen Winkel α einschliessen, dessen Öffnung gegen das distale Ende (3) gerichtet ist.

2. Frakturfixationsplatte (1) nach Anspruch 1, **dadurch gekennzeichnet, dass** der Winkel α < 175° ist und vorzugsweise im Bereich von 110° bis 160° liegt.

3. Frakturfixationsplatte (1) nach Anspruch 1 oder 2, **dadurch gekennzeichnet, dass** die beiden Flügel (5,6) kreiszylinderförmig gebogen sind.

4. Frakturfixationsplatte (1) nach einem der Ansprüche 1 bis 3, **dadurch gekennzeichnet, dass** die beiden Flügel (5,6) die gleiche Biegung, vorzugsweise die gleiche kreiszylinderförmige Biegung, aufweisen.

5. Frakturfixationsplatte (1) nach einem der Ansprüche 1 bis 4, **dadurch gekennzeichnet, dass** die Mittellinien (11,12) helixförmig gebogen sind.

6. Frakturfixationsplatte (1) nach einem der Ansprüche 1 bis 5, **dadurch gekennzeichnet, dass** der distale Abschnitt (18) des Körperabschnitts (2) eine Längsmittellinie (13) definiert, welche die Ebene, in welcher die gebogene Mittellinie (11) des rechten Flügels (5) liegt, unter einem Winkel β ≠ 90° schneidet.

7. Frakturfixationsplatte (1) nach Anspruch 6, **dadurch gekennzeichnet, dass** der Winkel β im Bereich zwischen 60° und 85° liegt.

8. Frakturfixationsplatte (1) nach einem der Ansprüche 1 bis 7, **dadurch gekennzeichnet, dass** der distale Abschnitt (18) des Körperabschnitts (2) eine Längsmittellinie (13) definiert, welche die Ebene, in welcher die gebogene Mittellinie (12) des linken Flügels (6) liegt unter einem Winkel γ ≠ 90° schneidet.

9. Frakturfixationsplatte (1) nach Anspruch 8, **dadurch gekennzeichnet, dass** der Winkel γ ≠ 90° im Bereich von 50° bis 80° liegt.

10. Frakturfixationsplatte (1) nach einem der Ansprüche 1 bis 9, **dadurch gekennzeichnet, dass** das proximale Ende (4) des Körperabschnitts (2) und die beiden Flügel (5,6) bündig sind.

11. Frakturfixationsplatte (1) nach einem der Ansprüche 1 bis 8, **dadurch gekennzeichnet, dass** der langgestreckten Körperabschnitt (2) eine Anzahl Gewindelöcher (14) aufweist, welche gegenüber der Längsmittellinie (13) versetzt angeordnet sind.

12. Frakturfixationsplatte (1) nach einem der Ansprüche 1 bis 11, **dadurch gekennzeichnet, dass** der Körperabschnitt (2) zusätzlich ein längliches Kompressionsloch (16) aufweist.

13. Frakturfixationsplatte (1) nach einem der Ansprüche 1 bis 12, **dadurch gekennzeichnet, dass** das Verhältnis zwischen der Länge L des linken Flügels (6) und der Länge I des rechten Flügels (5) der Bedingung L ≥ 1,2 l gehorcht.

14. Frakturfixationsplatte (1) nach einem der Ansprüche 1 bis 5 und 10 bis 12, **dadurch gekennzeichnet, dass** die Längsmittellinie (13) die Ebene, in welcher die gebogene Mittellinie des linken Flügels liegt, unter einem Winkel β_{LH} ≠ 90° schneidet.

15. Frakturfixationsplatte (1) nach einem der Ansprüche 1 bis 14, zur Behandlung von Knochenfrakturen des proximalen Humerus.

## Claims

1. A fracture fixation plate (1) for application to the proximal humerus, wherein
a) the fracture fixation plate (1) has an elongate body section (2) which has a free distal end (3) and a proximal end (4) to which there are adjoined, on each side, a right wing (5) having the center line (11) and a left wing (6) having the center line (12);
b) the body section (2) and the two wings (5, 6) are provided with a number of screw holes (7) for receiving bone-fastening elements (8), and the fracture fixation plate (1) has a bone contact face (9) and an opposite surface (10), and
c) the two wings (5, 6) have different lengths,
**characterized in that**
d) the two wings (5, 6) are curved; and
e) the extensions of the two curved center lines (11, 12) form an obtuse angle α the opening of which is directed towards the distal end (3).

2. The fracture fixation plate (1) according to claim 1, **characterized in that** the angle α < 175° and is preferably in the range from 110° to 160°.

3. The fracture fixation plate (1) according to claim 1 or 2, **characterized in that** the two wings (5, 6) have a circular cylindrical curvature.

4. The fracture fixation plate (1) according to any one of claims 1 to 3, **characterized in that** the two wings (5, 6) have the same curvature, preferably the same circular cylindrical curvature.

5. The fracture fixation plate (1) according to any one of claims 1 to 4, **characterized in that** the center lines (11, 12) have a helical curvature.

6. The fracture fixation plate (1) according to any one of claims 1 to 5, **characterized in that** the distal section (18) of the body section (2) defines a longitudinal center line (13) which intersects the plane in which the curved center line (11) of the right wing (5) lies, at an angle β ≠ 90°.

7. The fracture fixation plate (1) according to claim 6, **characterized in that** the angle β is in the range between 60° and 85°.

8. The fracture fixation plate (1) according to any one of claims 1 to 7, **characterized in that** the distal section (18) of the body section (2) defines a longitudinal center line (13) which intersects the plane in which the curved center line (12) of the left wing (6) lies, at an angle γ ≠ 90°.

9. The fracture fixation plate (1) according to claim 8, **characterized in that** the angle γ ≠ 90° is in the range from 50° to 80°.

10. The fracture fixation plate (1) according to any one of claims 1 to 9, **characterized in that** the proximal end (4) of the body section (2) and the two wings (5, 6) are flush.

11. The fracture fixation plate (1) according to any one of claims 1 to 8, **characterized in that** the elongate body section (2) has a number of threaded holes (14) which are arranged offset with respect to the longitudinal center line (13).

12. The fracture fixation plate (1) according to any one of claims 1 to 11, **characterized in that** the body section (2) additionally has an elongate compression hole (16).

13. The fracture fixation plate (1) according to any one of claims 1 to 12, **characterized in that** the ratio between the length L of the left wing (6) and the length l of the right wing (5) satisfies the condition L ≥ 1.2 l.

14. The fracture fixation plate (1) according to any one of claims 1 to 5 and 10 to 12, **characterized in that** the longitudinal center line (13) intersects the plane in which the curved center line of the left wing lies, at an angle β_{LH} ≠ 90°.

15. The fracture fixation plate (1) according to any one of claims 1 to 14 for treating bone fractures of the proximal humerus.

## Revendications

1. Plaque de fixation de fractures (1) destinée à être appliquée sur l'humérus proximal,
a) la plaque d'ostéosynthèse pour le traitement de fractures (1) présentant un segment de corps (2) allongé qui présente une extrémité distale (3) libre et une extrémité proximale (4) à laquelle respectivement latéralement une aile droite (5) se raccorde à la ligne médiane (11) et une aile gauche (6) à la ligne médiane (12) ;
b) le segment de corps (2) et les deux ailes (5, 6) étant munis d'un certain nombre de trous pour vis (7) destinés à recevoir des éléments de fixation d'os (8), et la plaque d'ostéosynthèse pour le traitement de fractures (1) présentant une surface de contact osseux (9) et une surface opposée (10), et
c) les deux ailes (5, 6) présentant une longueur différente,
**caractérisée en ce que**
d) les deux ailes (5, 6) sont courbées ; et
e) les prolongations des deux lignes médianes (11, 12) courbées forment un angle α obtus dont l'ouverture est dirigée vers l'extrémité distale (3).

2. Plaque de fixation de fractures (1) selon la revendication 1, **caractérisée en ce que** l'angle α est < 175° et se situe de préférence dans la plage de 110° à 160°.

3. Plaque de fixation de fractures (1) selon la revendication 1 ou 2, **caractérisée en ce que** les deux ailes (5, 6) sont courbées en forme de cylindre circulaire.

4. Plaque de fixation de fractures (1) selon l'une des revendications 1 à 3, **caractérisée en ce que** les deux ailes (5, 6) présentent la même courbure, de préférence la même courbure en forme de cylindre circulaire.

5. Plaque de fixation de fractures (1) selon l'une des revendications 1 à 4, **caractérisée en ce que** les lignes médianes (11, 12) sont courbées en forme d'hélice.

6. Plaque de fixation de fractures (1) selon l'une des revendications 1 à 5, **caractérisée en ce que** le segment distal (18) du segment de corps (2) définit une ligne médiane longitudinale (13) qui coupe selon un angle β ≠ 90° le plan dans lequel se situe la ligne médiane (11) courbée de l'aile droite (5).

7. Plaque de fixation de fractures (1) selon la revendication 6, **caractérisée en ce que** l'angle β se situe dans la plage entre 60° et 85°.

8. Plaque de fixation de fractures (1) selon l'une des revendications 1 à 7, **caractérisée en ce que** le segment distal (18) du segment de corps (2) définit une ligne médiane longitudinale (13) qui coupe sous un angle γ ≠ 90° le plan dans lequel se situe la ligne médiane (12) courbée de l'aile gauche (6).

9. Plaque de fixation de fractures (1) selon la revendication 8, **caractérisée en ce que** l'angle γ ≠ 90° se situe dans plage de 50° à 80°.

10. Plaque de fixation de fractures (1) selon l'une des revendications 1 à 9, **caractérisée en ce que** l'extrémité proximale (4) du segment de corps (2) et les deux ailes (5, 6) sont attachées.

11. Plaque de fixation de fractures (1) selon l'une des revendications 1 à 8, **caractérisée en ce que** le segment de corps (2) allongé présente un certain nombre de trous pour vis (14) qui sont disposés de façon décalée par rapport à la ligne médiane longitudinale (13).

12. Plaque de fixation de fractures (1) selon l'une des revendications 1 à 11, **caractérisée en ce que** le segment de corps (2) présente en plus un trou de compression (16) oblong.

13. Plaque de fixation de fractures (1) selon l'une des revendications 1 à 12, **caractérisée en ce que** le rapport entre la longueur L de l'aile gauche (6) et la longueur l de l'aile droite (5) respecte la condition L ≥ 1,2 I.

14. Plaque de fixation de fractures (1) selon l'une des revendications 1 à 5 et 10 à 12, **caractérisée en ce que** la ligne médiane longitudinale (13) coupe selon un angle β_{LH} ≠ 90° le plan dans lequel se situe la ligne médiane courbée de l'aile gauche.

15. Plaque de fixation de fractures (1) selon l'une des revendications 1 à 14, pour traitement sur l'humérus proximal.
